# EUROPEAN PATENT SPECIFICATION

(11) **EP 4 429 779 B1**
(45) Date of publication and mention of the grant of the patent: **01.07.2026**
(21) Application number: 21814693.4
(22) Date of filing: 09.11.2021
(51) Int. Cl.: A61Q 19/08, A61K 8/9789, A61K 8/49, A61K 8/60

(54) **EXTRACT OF YOUNG HIBISCUS FLOWERS AND COSMETIC USES**
EXTRAKT AUS JUNGEN HIBISKUSBLÜTEN UND KOSMETISCHE ANWENDUNGEN
EXTRAIT DE JEUNES FLEURS D'HIBISCUS ET UTILISATIONS COSMÉTIQUES

(43) Date of publication of application: 18.09.2024
(73) Proprietor: Societe Industrielle Limousine d'Application Biologique, 19240 Saint Viance (FR); ELC Management LLC, Melville NY 11747 (US)
(72) Inventor: PAUFIQUE, Jean, 19130 OBJAT (FR); PERNODET, Nadine, NEW YORK, Dix Hills 11746 (US); CHEN, Chia-Wen, NEW YORK, Eastchester 10709 (US); TRIVERO, Jacqueline Mary, NEW YORK, Putnam Valley 10579 (US); CORALLO, Krystle, NEW YORK, Huntington 11743 (US)
(74) Representative: Aquinov
(86) International application number: PCT/EP2021/081149
(87) International publication number: WO 2023/083437

(56) References cited:
- CN-A- 107 343 866

## Description

### Technical field

The invention relates to cosmetics, and in particular a specific cosmetic active ingredient obtained from young Hibiscus flowers according to claim 1. The invention also relates to the cosmetic use of this active ingredient for topical application on the skin, in particular for a skin antiaging effect.

### Background and summary of the invention

Skincare using natural products is increasingly sought after by consumers. Among these products, flower extracts are in particular commonly used in cosmetics.

One aim of the invention is to propose an extract of particular flowers that has a high cosmetic efficacy, and in particular that makes it possible simultaneously to promote skin regeneration, regulate the epigenetic factors responsible for skin aging, increase cell detoxification of the skin, limit skin inflammation and strengthen the skin immune barrier, for a skin antiaging effect.

To achieve this, the invention proposes to use young flowers, flowers that change color during the day, namely young *Hibiscus sinensis* flowers.

Chinese Hibiscus (*Hibiscus sinensis*) is an astonishing shrub that bears flowers with multiple and delicately creased petals. The use of these flowers is known in traditional Chinese medicine. The calyxes are in particular known for their detoxifying and blood temperature regulating action.

When they deploy their corolla in the morning, the flowers are stained a beautiful ivory white color. Over the course of the day, the petals become increasingly pink, and they are an intense pinkish red at the end of flowering.

According to the invention, the color change of the *Hibiscus sinensis* petals is closely linked to the lifetime of the flower, which is exposed to several environmental factors. In the early morning, the young flower blooms and bears white petals. During the day, the exposure to light and the outside temperature affects the flowers, which mature and turn pink. In the evening, the pinkish red corollas show the age of these venerable flowers, the lifetime of which does not exceed two days. This symphony of color has a strong impact on the behavior of pollinators, who can thus discriminate between young and old flowers and assess the availability of their floral reward. These color variations can be explained by the change in the chemical composition of the petals over the course of the day and the presence of different metabolites.

Surprisingly, according to the invention, the young white to light pink flowers have a more powerful antiaging activity than the older red or bright pink flowers.

The prior art, in particular in (Journal of Tropical Forest Science 21(4): 307-315 Antioxidant properties of Hibiscus: species variation, altitudinal change, coastal influence and floral colour change; SK Wong, YY Lim, EWC Chan) states that the antioxidant activity of extracts of *Hibiscus sinensis* petals is proportional to the color of the flower, and therefore proportional to the anthocyan content of the flower, and that as a result, the antioxidant effect of the petals of red flowers would be greater than that of young white to light pink flowers. Yet conversely, according to the invention, the white to light pink *Hibiscus sinensis* flowers have:
- molecules that act to combat skin aging, and that are present little or not at all in the red flowers, namely N-(1-deoxy-1-fructosyl)-proline and sarmentosin epoxide, and
- a greater skin antiaging efficacy than that of red flower extracts, which may even be nonexistent. See also CN107343866 A.

Thus, the invention relates to a cosmetic active ingredient comprising at least one extract obtained from *Hibiscus sinensis* flowers, said flowers are selected on their white to light pink color. Due espacially to this selection, the active ingredient can be charaterized in that it comprises:
- at least 2 ppm of N-(1-deoxy-1-fructosyl)-proline, and/or
- at least 145 ppm of sarmentosin epoxide.

The invention also relates to the cosmetic use on healthy skin of such extracts or a composition containing them, by topical application on the skin, in particular a cosmetic use to combat skin aging.

Lastly, the invention relates to cosmetic compositions comprising said extracts and a cosmetic treatment method for healthy skin for a skin antiaging effect.

The present invention is now described in detail.

### Detailed description of the invention

### Definitions

Within the meaning of the invention, "cosmetic active ingredient" or "active ingredient" refers to at least one molecule, preferably a set of molecules, having a cosmetic effect on the skin, i.e., having an effect on the skin cells.

Within the meaning of the invention, *"Hibiscus sinensis"* refers to all synonyms: *"Abelmoschus mutabilis", "Abelmoschus venustus", "Hibiscus immutabilis", "Hibiscus javanicus", "Hibiscus mutabilis"* and *"Ketmic mutabilis".*

Within the meaning of the invention, "white to light pink flowers" refers to white flowers only, or light pink flowers only, or a mixture of white and light pink flowers, the ratio of white flowers to light pink flowers being irrelevant.

"Ethanolic extract" refers to an extract for which the extraction step from the plant is done in ethanol or a water/ethanol solution. It does not relate to extracts done by an extraction step in another solvent, solvent which is subsequently evaporated, the pellet being re-dissolved in ethanol or a water/ethanol solution.

"Hydroglycolic extract" means that the extraction step from the plant is done in a water/butylene glycol solution. It does not relate to extracts done by an extraction in another solvent, solvent which is subsequently evaporated, the pellet being re-dissolved in a water/butylene glycol solution.

### Cosmetic active ingredient

The invention therefore relates to a cosmetic active ingredient comprising at least one extract obtained from *Hibiscus sinensis* flowers, comprising:
- at least 2 ppm of N-(1-deoxy-1-fructosyl)-proline; N-(1-deoxy-1-fructosyl)-proline is a glycosylated amino acid with molar mass 277 Da; and/or
- at least 145 ppm of sarmentosin epoxide; sarmentosin epoxide is a glycoside with molar mass 291 Da.

Said *Hibiscus sinensis* flowers having white to light pink color.

Preferably, the *Hibiscus sinensis* flowers extract(s) are hydroglycolic extract(s) of *Hibiscus sinensis* flowers.

Yet the hydroglycolic extracts of red *Hibiscus sinensis* flowers comprise little or none of these two molecules (less than 2 ppm of N-(1-deoxy-1-fructosyl)-proline and less than 145 ppm of sarmentosin epoxide).

Moreover, the extract(s) of white to light pink *Hibiscus sinensis* flowers present in the active ingredient according to the invention comprise few or no maturity marker molecules, which are, however, present in the red flowers, namely citric acid and another organic acid, with a structure close to citric acid.

Thus, preferably the extract(s) of *Hibiscus sinensis* flowers present in the active ingredient according to the invention comprises:
- less than 150 ppm of citric acid with a molar mass of 192 Da, and/or
- less than 60 ppm of an isomeric of citric acid with a molar mass of 192 Da.

Yet the hydroglycolic extracts of red *Hibiscus sinensis* flowers comprise these two molecules (more than 150ppm of citric acid and more than 60ppm of an another organic acid, with a structure close to citric acid).

Furthermore, preferably, the extract present in the active ingredient of the invention has at least one of the following features, preferably all of them:
- it comprises at least 50% carbohydrates by dry weight of the extract,
- it comprises at least 30% by dry weight of the carbohydrates present in the extract, are oligosaccharides and polysaccharides with molar masses of between 360 and 1,620 Da,
- it comprises minerals and/or proteins, preferably less than 20% minerals and/or less than 20% proteins, the percentages being given by dry weight of the extract,
- the proteins present in the extract are peptides with molar masses below 2,000 Da.

The sugar (carbohydrate) content in the extract can be determined using the DUBOIS method (Dubois M. et al., Analytical chemistry, 28, 3, 350-356, 1956). According to one preferred embodiment, the total sugar content of the extract is between 50 and 85% by dry weight of the extract.

The carbohydrates present in the extract are preferably at least in part monosaccharides and oligosaccharides with molar masses of between 360 Da and 1,620 Da (or a degree of polymerization greater than 2 and less than 9). Preferably, the oligosaccharides with molar masses of between 360 Da and 1,620 Da represent at least 30% by weight of the carbohydrates present in the extract, still more preferably at least 40%.

The carbohydrates present in the extract are preferably made up of fructose, saccharose and glucose. This simple sugar composition of the extract can be determined in particular by ion liquid chromatography.

Thus, the carbohydrate fraction of the extract according to the invention is preferably made up of fructose, saccharose and glucose in the form of oligosaccharides with molar masses of between 360 Da and 1,620 Da.

The active ingredient according to the invention may also comprise minerals and/or proteins and/or polyphenolic compounds.

The crude ash (mineral) content can be determined by weighing residues from the incineration of samples of the active ingredient according to the invention at 550°C in an electric muffle furnace. Preferably, the active ingredient according to the invention contains less than 20% minerals (ash rate) by dry weight of the extract.

The content in peptide compounds (proteins) in the active ingredient according to the invention may be determined by assaying the total nitrogen using the KJELDHAL method (reference: Official method of analysis of the A.O.C., 12th ed. W Horwitz, E.D., New-York, 15-60, 1975). Preferably, the active ingredient according to the invention contains less than 20% peptides. The molar mass of the peptides of the active ingredient according to the invention is less than 2,000 Da by dry weight of the extract.

The content in polyphenolic compounds may be determined using the following method. The polyphenolic compounds form in the presence of potassium ferricyanide of the colored compounds, detectable at 715 nm. The color intensity is proportional to the quantity of polyphenolic compounds. The readings are done from a gallic acid benchmark range of from 40 to 120 mg/L. The results obtained for the benchmarks make it possible to draw an optical density line as a function of the concentration, and the polyphenol level of the samples is read directly on said line. The polyphenolic compound level of the extract according to the invention is expressed in percentage of gallic acid equivalent relative to the dry content of the extract according to the invention. Preferably, the active ingredient according to the invention contains less than 2% of polyphenolic compounds, which are primarily phenolic acids, flavonoids or their glycoside derivatives.

The active ingredient according to the invention may assume a solid form or a liquid form.

When it assumes a liquid form, the active ingredient according to the invention is preferably exclusively made up of the extract as previously described. It generally assumes the form of a clear liquid, with a faint odor and a yellow color, preferably light yellow. It may, however, be colored and/or be bleached using any method known by one skilled in the art. Preservatives or stabilizers well known by one skilled in the art may be added to the extract according to the invention to form the active ingredient according to the invention.

When it assumes a solid form, in particular powder form, it is made up of the *Hibiscus sinensis* flower extract according to the invention and a carrier such as maltodextrins. Preferably, the extract represents at least 10% by weight of the active ingredient and the carrier no more than 90%.

### Method for obtaining the cosmetic active ingredient

The extract present in the active ingredient according to the invention can be obtained by any type of extraction method, except an ethanolic extract.

Preferably, the method according to the invention comprises a step for solubilization, in a water/butylene glycol mixture, of white to light pink color *Hibiscus sinensis* flowers.

Still more preferably, the method according to the invention it comprises carrying out the following steps:
- solubilizing the *Hibiscus sinensis* flowers powder in a water/butylene glycol mixture,
- separating the soluble and insoluble phases, to recover the soluble phase,
- filtrations,
- sterilizing filtration.

Clarification and/or bleaching and/or deodorizing steps may also be added.

The steps of the methods described above, considered individually, are typical in the field of active ingredient extractions from natural raw materials, and one skilled in the art is able to adjust the reaction parameters thereof based on his general knowledge.

### Uses of the the cosmetic active ingredient

The active ingredient according to the invention is particularly effective for a cosmetic treatment on healthy skin, therefore not therapeutic.

In particular, the active ingredient according to the invention is effective on healthy (nonpathological) skin, at once to promote skin regeneration, stimulate epigenetic factors, boost cell detoxification, limit skin inflammation and strengthen the skin immune barrier function.

The invention therefore also relates to the cosmetic use of a cosmetic active ingredient as previously described or a composition containing it, applied topically on healthy skin, in particular to combat skin aging.

During aging, the skin's ability to regenerate decreases. This results from a loss of the cells' ability to proliferate and migrate and is reflected by a change in the general appearance of the skin. Indeed, it is in particular noted that the structure of the epidermis changes during aging: older epidermises then have a smaller thickness relative to young epidermises. Advantageously, the active ingredient according to the invention, normalizes the thickness of the epidermis of older skin.
It increases cell proliferation (viewed via an increase in Ki-67 synthesis) on the one hand and cell migration on the other hand.

It thus increases cell metabolism and promotes tissue regeneration.

Furthermore, in order to limit skin aging directly, it is essential to act on multiple levels:
- maintaining hydration,
- preserving and strengthening the dermal matrix,
- activating longevity factors.

To be well hydrated, the skin must form a substantial water reservoir, then guarantee that it is properly distributed in the tissues. These functions are performed by two essential players that regulate the "water capital" of the skin:
- hyaluronic acid, which is a molecule with exceptional hydrating virtues. Indeed, it acts as a veritable "molecular sponge" capable of capturing up to 1,000 times its weight in water. Thus, its presence guarantees substantial reserves, primarily in the dermis, which contains 70% of the water from the skin. It is in this compartment that the fibroblasts will see to the production of this molecule, since they contain all of the enzymatic machinery necessary for the synthesis of hyaluronic acid (HAS, *hyaluronic acid synthases,* enzymes).
- the aquaporins, which are small channels integrated into the cell membranes. They allow the dynamic and efficient circulation of water. Aquaporin 3 is primarily present in the skin, especially in the membrane of the cells. The hydration level of the epidermis depends on the optimal distribution of these channels in all of the layers. By providing the flow of three billion molecules of water per second, they guarantee that the epidermis is correctly irrigated up to the surface.
Advantageously, the active ingredient according to the invention, is capable of stimulating the production of these two key players. It increases hyaluronic acid production and aquaporin 3 expression. Thus, the active ingredient according to the invention is capable of improving the hydration state of older skin.

The dermal matrix is one of the main targets of aging. Indeed, during this process, it experiences profound remodeling. This is in particular reflected by decreased expression of the various components of the extracellular matrix (ECM). These components include collagen, basic structural element of the ECM, as well as proteoglycans, such as versican. Other proteins play a major role in maintaining the ECM. Periostin is a necessary protein to maintain the operation of the collagen. Indeed, it influences the synthesis of the collagen fibrils by promoting their assembly. Vimentin is an intracellular protein that participates in the formation of intermediate filaments. It participates in maintaining the shape of the fibroblasts and the integrity of their cytoplasm. Fibulin 5 participates in generating elastic fibers by promoting the binding of elastin precursors. Lastly, integrin is a membrane receptor for collagen. It is described as regulating collagen synthesis and organization of the neo-synthesized extracellular matrix. It appears that during aging, all of these markers have a reduced expression/synthesis.
Advantageously, the active ingredient according to the invention is capable of stimulating the production of the components of the extracellular matrix. Thus, the active ingredient according to the invention is capable of stimulating the expression of collagen I, versican, periostin, vimentin, integrins α2 and fibulin 5. It also stimulates the production of procollagen I, precursor of collagen I.

To conclude, the skin has a "youth capital" that is correlated to the level of longevity markers, including NRF2 and FOXO3A. These two markers are recognized for their favorable effect on longevity. This effect in particular involves activation of the free radical stress response pathways or the inhibition of the entry to senescence.
Advantageously, the active ingredient according to the invention, is capable of stimulating the expression of longevity markers. It increases the expression of NRF2 and FOXO3A. Thus, the active ingredient according to the invention is capable of improving the longevity of older skin.

Beyond the genetic component, aging depends on an epigenetic component. Indeed, gene expression is conditioned by our environment. Epigenetic factors will incorporate these environmental changes and subsequently specifically activate or inhibit the expression of certain genes. Among these epigenetic factors, sirtuin 1 is the best known. It stabilizes chromatin, but it is also responsible for canceling the expression of certain genes, such as p53, that act negatively on longevity.
Advantageously, the active ingredient according to the is capable of stimulating the expression of an epigenetic factor. It increases the expression of SIRT-1.

Furthermore, aging results in part from an accumulation of oxidative damage. Our skin is equipped with different systems that allow it to protect itself and to adapt to these unfavorable conditions. Among these systems, the free radical enzymes (SOD1, thioredoxin, catalase) will help break down antioxidant molecules, while the stress response proteins HSP will keep the proteins in good working order.
Advantageously, the active ingredient according to the invention, is capable of stimulating the expression of factors involved in cell detoxification. It increases the expression of SOD, thioredoxin, catalase as well as molecular chaperones such as HSP72, HSP27 and HSP92. The HSPs, heat shock proteins or stress proteins, make up an important cell protection system. They act as chaperones and limit the distortion of the proteins in response to stress. The latter then remain fully functional. They promote the 3D structuring of the proteins and thus prevent them from being distorted, but also support the restoration of the incorrectly folded proteins.

The skin is subjected to different types of attacks daily. These may, *inter alia,* be microbial or solar. They cause a major inflammatory reaction that is reflected by the production of mediators such as prostaglandins E2 and interleukins 8 (IL-8). The latter may have major consequences on the skin physiology. Therefore, it is essential for the skin to protect itself against these inflammatory phenomena.

Advantageously, the active ingredient according to the invention, is capable of reducing inflammatory phenomena. It reduces the production of PGE2 and IL-8.

The epidermis actively participates in defending the skin by establishing an immune barrier, which involves a defense system. The keratinocytes have, on their surface, danger sensors that give them the ability to detect unwanted microorganisms. This function is performed by proteins from the TLR (Toll-Like Receptors) family. These TLR receptors have the ability to discriminate the presence of foreign microorganisms. The engagement of the TLR receptors of the keratinocytes activates several signaling cascades, and in particular the production of antimicrobial peptides. These key molecules include β-defensins (hBDs). Advantageously, the active ingredient according to the invention, acts on the immune barrier function. It activates the expression of the TLR-2 receptors and the hBD2.

Thus, the active ingredient according to the invention made in particular be used to:
- promote skin regeneration, in particular by stimulating epidermal renewal, synthesis of the key marker for Ki-67 proliferation and cell migration.
- combat skin aging, in particular by regulating and activating the key mediators of hydration (aquaporin 3, hyaluronic acid), stimulating the compounds of the extracellular matrix such as collagen I, versican, periostin, vimentin, integrin α 2 and fibulin 5 and also by stimulating the proteins involved in cell longevity such as NRF2 and FOXO3A.
- regulate epigenetic factors, and in particular SIRT-1 (sirtuin 1), which plays an essential role in controlling the expression of genes such as p53, which acts negatively on longevity.
- activate cell detoxification by regulating free radical enzymes (SOD1, thioredoxin and catalase) and Heat shock protein stress response factors (HSP72, HSP27 and HSP90).
- limit skin inflammation by decreasing PGE2 and interleukin IL-8 production during various attacks.
- regulate the immune barrier function by stimulating the production of the TLR2 danger receptor and antimicrobial peptides of the skin, in particular hBD2.

The active ingredient according to the invention therefore has a multi-functionality to improve the state of healthy skin, in particular older healthy skin, particularly to improve the skin parameters of said skin particularly for an antiaging effect.

The active ingredient according to the invention is preferably used in compositions comprising a cosmetically acceptable medium. It involves compositions in different dosage forms, suitable for topical application on the skin.

These compositions may in particular assume the form of oil-in-water emulsions, water-in-oil emulsions, multiple emulsions (Water/Oil/Water or Oil/Water/Oil) that may optionally be micro-emulsions or nano-emulsions, or in the form of solutions, suspensions, hydrodispersions, aqueous gels or powders. They may be more or less fluid and have the appearance of creams, emulsions or gels or any other appearance of skincare cosmetics.

It may involve compositions comprising at least 0.1% of a liquid active ingredient according to the invention, preferably between 0.5 and 10% or comprising at least 0.01% of a solid active ingredient (preferably powder) according to the invention.

These compositions comprise, aside from the active ingredient, a physiologically acceptable and preferably cosmetically acceptable medium, i.e., which does not cause feelings of discomfort for the user such as redness, tightness or stinging.

The compositions according to the invention may contain, as additive, at least one compound chosen from among:
- oils, which may in particular be chosen from among the linear or cyclic, volatile or nonvolatile oils,
- waxes, such as ozokerite, polyethylene wax, beeswax or carnauba wax,
- silicone elastomers,
- surfactants, preferably emulsifiers, whether non-ionic, anionic, cationic or amphoteric,
- co-surfactants, such as linear fatty alcohols,
- thickeners and/or gelling agents,
- humectants, such as polyols like glycerin,
- dyes, preservatives, fillers,
- tensor agents,
- sequestering agents,
- fragrances,
- and mixtures thereof, this list not being exhaustive.
Examples of such additives are in particular cited in the CTFA Dictionary *(International Cosmetic Ingredient Dictionary and Handbook* published by the *Personal Care Product Council).*

Of course, one skilled in the art will choose any additional compounds, whether or not they are active, and their quantity, such that the advantageous properties of the mixture are altered little or not at all by the considered addition.

These compositions are in particular intended to be used on healthy skin, in particular older healthy skin, for a skin antiaging effect.

The invention also targets a (non-therapeutic) cosmetic method, namely a cosmetic method for treating healthy skin for a skin antiaging effect, which consists of the topical application on healthy skin of a composition comprising an active ingredient according to the invention.

Preferably, the composition comprising the cosmetic active ingredient according to the invention is applied at least once per day for at least 15 days.

In order to illustrate these cosmetic effects on the quality of the skin, in particular on the properties of the skin, the following examples and test results are presented below.

### Examples

### Example 1: example of active ingredient according to the invention in liquid form derived from white to light pink flowers

The active ingredient is obtained by carrying out the following steps:
- solubilizing the white to light pink color *Hibiscus sinensis* flowers powder in a water/butylene glycol mixture,
- separating the soluble and insoluble phases, to recover the soluble phase,
- filtrations,
- sterilizing filtration.
The obtained active ingredient in particular has the following analytic characteristics:
- clear light yellow liquid, slight odor,
- dry content of 13 g/L,
- total polyphenol content of 0.15 g/L, or 0.5% relative to the dry matter,
- presence of 5.4 ppm of N-(1-deoxy-1-fructosyl)-proline, and of 163.5 ppm of sarmentosin epoxide,
- presence of 64 ppm of citric acid and 27 ppm of an isomer organic acid of citric acid.

The phenolic compounds were identified in example 1 by LC-MS/MS at 280 nm. The components of the extract are cited in Table 1 below.

**[Table 1]**

| Family | Attribution |
|---|---|
| **Anthocyanin** | Cyanidin-3-Glucoside * |
| | Cyanidin-3-Sambubioside * |
| | Cyanidin-3-Sambubioside derivative |
| | Cyanidin-3-rutinoside -5-glucoside |
| **Flavonoid** | Hibiscetin |
| **Flavonoid glycoside** | Malvidin-3-(6-malonylglucoside) |
| | Quercitrin * |
| | Tiliroside * |
| **Flavonol** | Kaempferol derivative |
| | Rutin * |
| | Kaempferol-3-O-rutinoside derivative |
| | Isoquercetin derivative |
| | Isoquercetin |
| | Kaempferol-3-O-rutinoside * |
| | Kaempferol * |
| **Flavonol glycoside** | Quercitrin-3,4'-diglucoside derivative |
| | Quercitrin-3,4'-diglucoside * |
| | Mutabiloside and derivative |
| | Quercetin-3-sambubioside and derivative |
| | Guajaverin * |
| | Trifolin |
| **Hydroxycinnamic acid** | Caffeic acid derivative |
| | Caffeic acid * |
| | Ferulic acid derivative |
| | Ferulic acid * |
| **Phenolic acid** | Protocatechuic acid and derivative |
| **phenolic acid derivative** | 2-O-caffeoylglucaric acid and derivative |
| **phenolic acid glycoside** | D-Glucopyranose, 6-(2-hydroxybenzoate) |
| **phenolic glycoside** | Mudanoside A |
| | Dopaol β-D-glucoside |
| | Caffeic acid 3-O-glucuronide |
| | β-D-Glucopyranose, 1-(4-hydroxy-3,5-dimethoxybenzoate) |
| | Caffeic acid 3-O-glucuronide |
| | Erigeside C |
| | 5-O-(trans-feruloyl)-L-arabinofuranose |

The polyphenolic compounds of the extract according to the invention of example 1 are primarily phenolic acids, flavonoids and their glycoside derivatives. The mutabiloside derivatives and the cyanidin-3-sambubioside are used as quality markers for the *Hibiscus* species.

### Example 2: example of active ingredient according to the invention in liquid form

### derived from white flowers

The active ingredient is obtained by carrying out the following steps:
- solubilizing the white *Hibiscus sinensis* flowers powder in a water/butylene glycol mixture,
- separating the soluble and insoluble phases, to recover the soluble phase,
- filtrations,
- sterilizing filtration.
The obtained active ingredient in particular has the following analytic characteristics:
- clear light yellow liquid, slight odor,
- dry content of 24.5 g/L,
- total polyphenol content of 0.17 g/L, or 0.7% relative to the dry matter,
- presence of 3.2 ppm of N-(1-deoxy-1-fructosyl)-proline, and of 175.6 ppm of sarmentosin epoxide
- presence of 135 ppm of citric acid and 40 ppm of an isomer organic acid of citric acid.

### Example 3: example of active ingredient according to the invention in liquid form

### derived from light pink flowers

The active ingredient is obtained by carrying out the following steps:
- solubilizing the light pink *Hibiscus sinensis* flowers powder in a water/butylene glycol mixture,
- separating the soluble and insoluble phases, to recover the soluble phase,
- filtrations,
- sterilizing filtration.
The obtained active ingredient has the following analytic characteristics:
- clear light yellow liquid, slight odor,
- dry content of 19.8 g/L,
- total polyphenol content of 0.17 g/L, or 0.9% relative to the dry matter,
- presence of 2.4 ppm of N-(1-deoxy-1-fructosyl)-proline, and of 147.4 ppm of sarmentosin epoxide
- presence of 92 ppm of citric acid and 47 ppm of an isomer organic acid of citric acid.

### Example 4: example of extract derived from red flowers (outside invention)

The ingredient is obtained by carrying out the following steps:
- solubilizing the red *Hibiscus sinensis* flowers powder in a water/butylene glycol mixture,
- separating the soluble and insoluble phases, to recover the soluble phase,
- filtrations,
- sterilizing filtration.
The red flower extract has the following analytic characteristics:
- clear light yellow liquid, slight odor,
- dry content of 22.0 g/L,
- total polyphenol content of 0.19 g/L, or 0.9% relative to the dry matter,
- presence of 1.5 ppm of N-(1-deoxy-1-fructosyl)-proline, and of 120.0 ppm of sarmentosin epoxide,
- presence of 193 ppm of citric acid and 85 ppm of an isomer organic acid of citric acid.

### Example 5: example of extract derived from pink flowers (outside invention)

The ingredient is obtained by carrying out the following steps:
- solubilizing the pink color *Hibiscus sinensis* flowers powder in a water/butylene glycol mixture,
- separating the soluble and insoluble phases, to recover the soluble phase,
- filtrations,
- sterilizing filtration.
The pink flower extract has the following analytic characteristics:
- yellow limpid liquid, weak odor,
- dry content of 20.7 g/L,
- total polyphenol content of 0.21 g/L, or 1.0% relative to the dry matter,
- less than 2 ppm of -(1-deoxy-1-fructosyl)-proline, and of 145 ppm of sarmentosin epoxide
- more than 150 ppm of citric acid and more than 60 ppm of an isomer organic acid of citric acid.

### Tests and Results

### I. Study of the effect of an active ingredient according to the invention on skin regeneration

The effect of the active ingredient according to the invention on skin regeneration was evaluated using several studies:
- on the morphological changes to older reconstructed skin, the thickness of the epidermis,
- on cell proliferation in older reconstructed skin, by evaluating the Ki-67 marker,
- on the migration of human fibroblasts.
The effect of an extract derived from red flowers (example 4) was also evaluated on cell migration for comparison.

### I.1. Effect of the active ingredient according to the invention on skin morphology

The purpose of this study is to evaluate the effect of the active ingredient according to the invention on the morphology of reconstructed skin done from older cells.
The action of the invention was studied on the morphological changes, including the thickness of the epidermis, by HES staining.

The operating protocol is described as follows:
On D0: the older or young human keratinocytes are seeded on a lattice respectively containing older or young human fibroblasts and incubated at 37°C.
During several days of treatment, the culture medium is eliminated and replaced by medium containing an active ingredient according to the invention. The reconstructed skin is next incubated at 37°C.
After several days of treatment, the reconstructed skin is recovered, which is then fixed, dehydrated and included in paraffin. Sections are taken using a microtome.
The morphological analysis is done by Hematoxylin Eosin Safran (HES) staining, and the viewing is done by microscope coupled to an image analysis system.
The thickness of the epidermis is measured on the histological sections.

The results are summarized in Table 2.

**[Table 2]**

| | **Measurement of the thickness of the epidermis (µm)** | **Capacity to improve the thickness of the epidermis (%)** |
|---|---|---|
| **Young reconstructed skin** | | |
| Control | 76 | |

| **Older reconstructed skin** | | |
|---|---|---|
| Control | 60 | |
| Active ingredient of example 1 0.25% | 68 | 50% |
| Active ingredient of example 1 0.50% | 74 | 88% |

One can see that the older reconstructed skin has a thinner epidermis (-21%) than the young reconstructed skin.
Tested at 0.5% on older reconstructed skin, the active ingredient according to the invention significantly improves the thickness of the epidermis by 88%.

### 1.2 Effect of the active ingredient according to the invention on Ki-67 synthesis

The purpose of this study is to evaluate the effect of the active ingredient according to the invention on a reconstructed skin model done from older cells. Ki-67 is among key markers for cell proliferation; it is present in the nucleus of the proliferative cells.

The action of the invention on cell proliferation has been studied by immunohistochemical marking of Ki-67.

The operating protocol is described as follows:
On D0: the older or young human keratinocytes are seeded on a lattice respectively containing older or young human fibroblasts and incubated at 37°C.
During several days of treatment, the culture medium is eliminated and replaced by medium containing the active ingredient according to the invention. The reconstructed skin is next incubated at 37°C.
After several days of treatment, the reconstructed skin is recovered, which is then fixed, dehydrated and included in paraffin. Sections are taken using a microtome. Their analysis is next done by immunohistochemical marking using a primary antibody (Ki-67 polyclonal antibody) and a secondary antibody (IgG antibody coupled with Alexa Fluor^{®} 488). The viewing is done by microscope coupled to an image analysis system.
The number of positive Ki-67 cells is determined manually in each image.

The results are summarized in Table 3.

**[Table 3]**

| | **Positive Ki-67 cells (%)** | **Capacity to improve Ki-67 synthesis (%)** |
|---|---|---|
| **Young reconstructed skin** | | |
| Control | 51 | |

| **Reconstructed older skin** | | |
|---|---|---|
| Control | 32 | |
| Active ingredient of example 1 0.25% | 39 | 37% |
| Active ingredient of example 1 0.50% | 42 | 53% |

One can see that the older reconstructed skin expresses less Ki-67 (-37%) compared with the young reconstructed skin.
Tested at 0.5% on older reconstructed skin, the active ingredient according to the invention significantly improves the synthesis of the cell proliferation marker, Ki-67, by 53%.

### 1.3 Effect of the active ingredient according to the invention on cell migration

The purpose of these studies is to evaluate the effect of the active ingredient according to the invention or an extract of red flowers on the migration of human fibroblasts.
Cell migration is the ability of certain cells to move. *In vitro,* it is evaluated by the number of cells covering a surface that is initially empty (Kim et al., 2015; Lampugnani, 1999). The migratory capacity of the cells is reduced with age (Boulter et al., 2013).
Their action was studied on a human fibroblast culture derived from older donors.

The operating protocol of the studies is described as follows:
On D0, the human fibroblasts are seeded and incubated at 37°C.
After several days of culture, the insert is removed from the culture chamber, creating a wound. The cells are treated with an active ingredient according to the invention or the red flower extract or a solution of N-(1-deoxy-1-fructosyl)-proline then incubated at 37°C. After several days of culture, the cells are stained with a crystal violet solution at 0.5% (M/V). The viewing is done with an AxioCam MRc camera with microscope coupled to an image analysis system.
The surface ratio occupied by the cells is calculated automatically using software.

The results obtained are summarized in Tables 4 and 5.

**[Table 4]**

| | **Occupied surface ratio (UA)** | **Cell migration (%)** |
|---|---|---|
| **Young fibroblast** | | |
| Control | 0.466 | |

| **Older fibroalasts** | | |
|---|---|---|
| Control | 0.290 | |
| Active ingredient of example 1 (white to light pink flowers) 1% | 0.351 | +21% |
| Active ingredient of example 2 (white flowers) 1% | 0.358 | +24% |
| Active ingredient of example 3 (light pink flowers) 1% | 0.346 | +20% |
| Extract of example 4 (red flowers) 1% | 0.236 | 0% |

One can see that the older fibroblasts have a significantly lower migration capacity (-38%) than that of the young fibroblasts.
Tested at 1% on older human fibroblasts, the red flower extract does not make it possible to activate the cell migration, while the active ingredients according to the invention, whether from white to light pink flowers, white flowers or light pink flowers, significantly increase the cell migration respectively by 21%, 24% and 20%.

This result clearly indicates the significance of choosing flowers on the desired efficacy.

**[Table 5]**

| | **Occupied surface ratio (UA)** | **Cell migration (%)** |
|---|---|---|
| **Young fibroblasts** | | |
| Control | 0.496 | |

| **Older fibroblasts** | | |
|---|---|---|
| Control | 0.275 | |
| Active ingredient of example 1 (white to light pink flowers) 1% | 0.329 | +20% |
| N-(1-deoxy-1-fructosyl)-proline 1.5 ppm | 0.266 | 0% |
| N-(1-deoxy-1-fructosyl)-proline 5ppm | 0.328 | +19% |

The results obtained with N-(1-deoxy-1-fructosyl)-proline indeed show that a content of 2 ppm participates in obtaining the expected effect on cell migration.

Advantageously, the active ingredient according to the invention, derived from white to light pink flowers, is therefore capable of stimulating skin regeneration. In particular, when it is applied on older skin, it:
- improves the morphology of the epidermis,
- increases Ki-67 expression,
- increases cell migration.

Thus, the active ingredient according to the invention, derived from white to light pink flowers, promotes the regeneration of older skin.

### II. Effect of the active ingredient according to the invention on skin aging

### II.1 Effect of the active ingredient according to the invention on skin hydration mediators

The effect of the active ingredient according to the invention on skin hydration mediators was evaluated using two studies
- on reconstructed older skin, by measuring hyaluronic acid synthesis,
- on older human fibroblasts, by evaluating the expression of aquaporin 3.

The effect of a red flower extract was also evaluated on the expression of aquaporin 3 for comparison.

### II.1.1. Effect of the active ingredient according to the invention on hyaluronic acid synthesis

The aim of this study is to determine the capacity of the active ingredient according to the invention to stimulate hyaluronic acid synthesis in reconstructed skin prepared with older cells.

The operating protocol is described as follows:
On D0: the older or young human keratinocytes are seeded on a lattice respectively containing older or young human fibroblasts and incubated at 37°C.
During several days of treatment, the culture medium is eliminated and replaced by medium containing the active ingredient according to the invention. The reconstructed skin is next incubated at 37°C.
After several days of treatment, the reconstructed skin is recovered, then fixed, dehydrated and included in paraffin. Sections are taken using a microtome.
A biotinylated protein having a strong affinity for hyaluronic acid (Hyaluronic Acid Binding Protein) and a secondary antibody are used to view the synthesis of the hyaluronic acid, in fluorescent green. A quantitative analysis of the images is done using the Matlab^{®} software.

The obtained results are summarized in Table 6.

**[Table 6]**

| | **Hyaluronic acid synthesis** | **Capacity to increase the hyaluronic acid synthesis** |
|---|---|---|
| | **(x10⁶ UA)** | **(%)** |
| **Young reconstructed skin** | | |
| Control | 330 | |

| **Reconstructed older skin** | | |
|---|---|---|
| Control | 218 | |
| Active ingredient of example 1 0.25% | 243 | +22% |
| Active ingredient of example 1 0.50% | 289 | +63% |

One can see that the older reconstructed skin has a reduced hyaluronic acid synthesis capacity (-34%), compared with the young reconstructed skin.
Tested at 0.5%, the active ingredient according to the invention significantly increases the synthesis of hyaluronic acid in older reconstructed skin by 63%.

### II.1.2. Effect of the active ingredient according to the invention on aquaporin 3 expression

The aim of this study is to determine the capacity of the active ingredient according to the invention or the red flower extract to increase aquaporin 3 expression in older skin cells.

Young human fibroblasts (P7) and older human fibroblasts (P24) are cultivated in a culture medium containing little or no active ingredient according to the invention or red flower extract.

After at least one day of treatment, the fibroblasts are collected and the whole RNAs are extracted. The mRNAs of the aquaporin 3 are analyzed in parallel with the mRNAs of RSP18. The quantification of the florescence incorporation is measured continuously using a thermocycler and the relative quantification is done using software.

The obtained results are summarized in Table 7.

**[Table 7]**

| | **Aquaporin 3 expression (%)** | **Ability to restore aquaporin 3 expression (%)** |
|---|---|---|
| **Young fibroblasts** | | |
| Control | 100 | |

| **Older fibroblasts** | | |
|---|---|---|
| Control | 46 | |
| Active ingredient of example 1 (white to light pink flowers) 0.25% | 68 | 41% |
| Active ingredient of example 2 (white flowers) 0.25% | 64 | 33% |
| Active ingredient of example 3 (light pink flowers) 0.25% | 58 | 22% |
| Extract of example 4 (red flowers) 0.25% | 42 | 0 |

One can see that the older fibroblasts have a reduced capacity to express aquaporin 3 relative to the young fibroblasts (-64%).
Tested at 0.25%, the active ingredients according to the invention increase the expression of aquaporin 3 from 22% to 41% in the older fibroblasts.
Moreover, one can see that the red flower extract has no action on the expression of aquaporin 3.
These results indicate the significance of the youngness of the flowers on the desired efficacy.

Advantageously, the active ingredient according to the invention, due to the presence of white to light pink flowers, is therefore capable of stimulating the production of these two key players. In particular, when it is applied on older skin, it increases:
- hyaluronic acid production,
- aquaporin 3 expression.

Thus, the active ingredient according to the invention, due to the presence of white to light pink flowers, improves the hydration of older skin.

### II.2. Effect of the active ingredient according to the invention on the dermal matrix

The effect of the active ingredient according to the invention on the markers of the dermal matrix was evaluated using several studies:
- on older human fibroblasts by passage owing to the measurement of the expression of different components of the matrix (collagen I, versican);
- on human fibroblasts derived from older donors owing to the measurement of the expression of different components of the matrix (periostin, vimentin, integrin α2, fibulin 5);
- on reconstructed older skin, by evaluating the synthesis of procollagen I.
The effect of a red flower extract was also compared in the study of the expression of collagen I and versican.

### II.2.1. Effect of the active ingredient according to the invention on collagen I and versican expression

The aim of this study is to determine the capacity of the active ingredient according to the invention or the red flower extract to increase collagen I and versican expression in older skin cells.

The extracellular matrix is a complex structure formed by a very organized network of fibers, in particular collagen fibers. Collagen I is a major structural component of these fibers. Beyond collagen fibers, the dermis is also made up of proteoglycans, among which versican plays an important role. It is described as being necessary to maintain skin firmness.

The operating protocol is described as follows: Young human fibroblasts (P7) and older human fibroblasts (P24) are cultivated in a culture medium containing little or no active ingredient according to the invention or red flower extract.
After at least one day of treatment, the fibroblasts are collected and the whole RNAs are extracted. The mRNAs of collagen I and versican are analyzed in parallel with the mRNAs of RSP18. The quantification of the florescence incorporation is measured continuously using a thermocycler and the relative quantification is done using software.

The results are summarized in Table 8.

**[Table 8]**

| | **Expression of collagen I (%)** | **Capacity to restore collagen I expression (%)** | **Expression of versican (%)** | **Capacity to restore versican expression (%)** |
|---|---|---|---|---|
| **Young fibroblasts** | | | | |
| Control | 100 | | 100 | |

| **Older fibroblasts** | | | | |
|---|---|---|---|---|
| Control | 65 | | 73 | |
| Active ingredient of example 1 (white to light pink flowers) 0.25% | 90 | +71% | 88 | +56% |
| Active ingredient of example 2 (white flowers) 0.25% | 95 | +86% | 89 | +59% |
| Active ingredient of example 3 (light pink flowers) 0.25% | 82 | +49% | / | / |
| Extract of example 4 (red flowers) 0.25% | 64 | 0 | 51 | 0 |

The older fibroblasts express less collagen I (-35%) and versican (-27%) than the young fibroblasts.
Tested 0.25% on older human fibroblasts, the red flower extract does not restore collagen I expression, while the active ingredients of white to light pink flowers increase collagen I expression by older fibroblasts from 49% to 86%.
Tested 0.25% on older human fibroblasts, the red flower extract does not restore versican expression, while the active ingredients of white to light pink flowers increase versican expression by older fibroblasts respectively by 56% and 59%.

These results indicate the significance of the youngness of the flowers on the desired efficacy.

### 11.2.2. Effect of flower harvesting time on the content of active molecules to deliver skin benefits

The aim of this study is to observe the effect of flower harvesting time on the content of active molecules to deliver skin benefits such as increase procollagen I production. The biological activity of 3 different extracts coming from the same Hibiscus Sinensis species but harvested at different times over the period of its flower lifetime, has been evaluated. The biological activity evaluated is the ability to support skin cells in producing collagen as it is critical for skin firmness, lifting and to combat lines and wrinkles.

The operating protocol is described as follows.

Normal Human Dermal Fibroblasts (NHDF) from a 62 years old donor were plated onto 6 well plates at a density of 50,000 cells/well and incubated at standard conditions (37°C/ 5% CO₂) overnight. Fibroblasts were grown in DMEM media supplemented with 10% Bovine Calf Serum and 1% Penicillin Streptomycin.

The samples were treated with active ingredients of exemple 2, 5 and 4 at 0.1%, 0.5%, or 1% in fully supplemented media. The plates were incubated at standard conditions (37°C/ 5% CO₂) for 96 hours. Following incubation, supernatants were harvested and stored at -80°C.

After 96 hours incubation and supernatants were collected, cell counts were performed for cell viability. Each sample was washed once with PBS and then administered 0.25% trypsin. After cells detached from the 6 well plates, media was administered to each sample to neutralize the trypsin. Cell counts were done with the Vicell.

Collagen I production was assessed using the Pro Collagen Type I ELISA. The optical density (OD) was measured at 450 nm.

Statistical analysis was performed on all samples using one-way ANOVA with a post test of Dunnett's in Graphpad InStat. Furthermore, error bars shown depict standard error as calculated by formula in Excel. (where ** p<0.01).

The results are summarized in Table 9: Procollagen I Production in comparison to control normalized to the averaged cell count. Active ingredient of example 2 (white flowers) at 1% shows the largest increase in procollagen I production in comparison to active ingredient of example 5 (white to light pink flowers) and active ingredient of example 4 (red flowers).

**[Table 9]**

| | **Capacity to product procollagen I (%)** |
|---|---|
| Control | - |
| Active ingredient of example 2 (white flowers) 0.1% | 34% |
| Active ingredient of example 2 (white flowers) 0.5% | 98% |
| Active ingredient of example 2 (white flowers) 1% | 183% |
| Active ingredient of example 5 (pink flowers) 0.1% | 21% |
| Active ingredient of example 5 (pink flowers) 0.5% | 32% |
| Active ingredient of example 5 (pink flowers) 1% | 34% |
| Active ingredient of example 4 (red flowers) 0.1% | 6% |
| Active ingredient of example 4 (red flowers) 0.5% | 28% |
| Active ingredient of example 4 (red flowers) 1% | 78% |

These results shown the critical importance of harvesting time when it comes to plants extracts and this precision is essential in order to deliver the best performance to the skin. 3 fractions of same flower species were extracted with same process from flowers harvested either at early time, medium or late time in the flower lifetime and showed that early harvesting is essential to increase collagen production in mature skin cells.

### II.2.3. Effect of the active ingredient according to the invention on the expression of different markers of the dermal matrix

The aim of this study is to determine the capacity of the active ingredient according to the invention to increase the expression of periostin, vimentin, integrin α2 and fibulin 5 in older skin cells.
Periostin is a necessary protein to maintain the operation of the collagen. Indeed, it influences the synthesis of the collagen fibrils by promoting their assembly. Vimentin is an intracellular protein that participates in the formation of intermediate filaments. It participates in maintaining the shape of the fibroblasts and the integrity of their cytoplasm. Fibulin 5 participates in generating elastic fibers by promoting the binding of elastin precursors. Lastly, integrin is a membrane receptor for collagen. It is described as regulating collagen synthesis and organization of the neo-synthesized extracellular matrix.

The operating protocol is described as follows:
Normal human fibroblasts from young donors (21 years old) or older donors (68 years old) are cultivated in a culture medium containing little or no active ingredient according to the invention.
After at least one day of contact, the fibroblasts are collected and the whole RNAs are extracted. The mRNAs of periostin, vimentin, integrin α2 and fibulin 5 are analyzed in parallel with the mRNAs of RSP18. The quantification of the florescence incorporation is measured continuously using a thermocycler and the relative quantification is done using software.

The results are summarized in Table 10.

**[Table 10]**

| | **Expression of periostin (%)** | **Capacity to restore periostin expression (%)** | **Expression of vimentin (%)** | **Capacity to restore vimentin expression (%)** | **Expression of integrin α2 (%)** | **Capacity to restore integrin α2 expression (%)** | **Expression of fibulin 5 (%)** | **Capacity to restore fibulin 5 expression (%)** |
|---|---|---|---|---|---|---|---|---|
| **Young fibroblasts** | | | | | | | | |
| Control | 100 | | 100 | | 100 | | 100 | |

| **Older fibroblasts** | | | | | | | | |
|---|---|---|---|---|---|---|---|---|
| Control | 55 | | 89 | | 79 | | 79 | |
| Active ingredient of example 10.25% | 68 | +71 | 96 | +64 | 110 | +148 | 85 | +29 |

The older fibroblasts express less periostin (-45%), vimentin (-11%), integrin α2 (-21%) and fibulin 5 (-21%) than the young fibroblasts.
Tested at 0.25% on older human fibroblasts, the active ingredient according to the invention makes it possible to restore periostin, vimentin, integrin α2 and fibulin 5 expression of older fibroblasts respectively 71%, 64%, 148% and 29%.

### II.2.4. Effect of the active ingredient according to the invention on the synthesis of procollagen I

The effect of the active ingredient according to the invention on procollagen I synthesis was evaluated using a study, on a reconstructed skin model done from older cells.

Procollagen I is the precursor of collagen I, which is a major structural component of the dermal matrix.

The operating protocol is described as follows:
On D0: the older or young human keratinocytes are seeded on a lattice respectively containing older or young human fibroblasts and incubated at 37°C.
During several days of treatment, the culture medium is eliminated and replaced by medium containing the active ingredient according to the invention. The reconstructed skin is next incubated at 37°C.
After several days of treatment, the reconstructed skin is recovered, which is then fixed, dehydrated and included in paraffin. Sections are taken using a microtome.
Their analysis is next done by immunohistochemical marking using a primary antibody (procollagen I antibody) and a secondary antibody (IgG antibody coupled with Alexa Fluor 488). The viewing is done by microscope coupled to an image analysis system.
The procollagen I synthesis is proportional to the intensity of the fluorescence (green color) present on the reconstructed skin.

The results are summarized in Table 11.

**[Table 11]**

| | **Synthesis of procollagen I (x10⁵ UA)** | **Capacity to improve procollagen I synthesis (%)** |
|---|---|---|
| **Young reconstructed skin** | | |
| Control | 155 | |

| **Reconstructed older skin** | | |
|---|---|---|
| Control | 113 | |
| Active ingredient of example 1 0.25% | 134 | +50% |
| Active ingredient of example 1 0.50% | 144 | +74% |

One can see that the older reconstructed skin synthesizes less procollagen I than the young reconstructed skin (-27%).
Tested at 0.5% on older reconstructed skin, the active ingredient according to the invention allows a significant improvement of the synthesis of procollagen I by 74%.

### II.3 Effect of the active ingredient according to the invention on longevity

The effect of the active ingredient according to the invention on longevity was evaluated using a study on older human fibroblasts, by evaluating the expression of NRF2 and FOXO3A.
NRF2 is described as the aging regulator. Indeed, when its expression is increased, this factor induces various pathways promoting longevity, including the free radical stress response pathways. FOXO3A is a central transcription factor. In its absence, it describes that cell senescence is favored.

The aim of this study is to determine the capacity of the active ingredient according to the invention to increase NRF2 and FOXO3A expression in older skin cells.

The operating protocol is described as follows:
Normal human fibroblasts from young donors (21 years old) or older donors (68 years old) are cultivated in a culture medium containing little or no active ingredient according to the invention.
After at least one day of contact, the fibroblasts are collected and the whole RNAs are extracted. The mRNAs of NRF2 and FOXO3A are analyzed in parallel with the mRNAs of RSP18. The quantification of the florescence incorporation is measured continuously using a thermocycler and the relative quantification is done using software.

The results are summarized in Table 12.

**[Table 12]**

| | **Expression of NRF2 (%)** | **Capacity to restore NRF2 expression (%)** | **Expression of FOXO3A (%)** | Capacity to **restore FOXO3A expression (%)** |
|---|---|---|---|---|
| **Young fibroblasts** | | | | |
| Control | 100 | | 100 | |

| **Older fibroblasts** | | | | |
|---|---|---|---|---|
| Control | 82 | | 67 | |
| Active ingredient of example 1 0.25% | 120 | +211% | 91 | +73% |

One can see that the capacity of the older fibroblasts to express NRF2 and FOXO3A is decreased respectively by -18% and -33% relative to young fibroblasts.
Tested at 0.25%, the active ingredient according to the invention makes it possible to increase the expression of NRF2 and FOXO3A respectively by 211% and 73% in the older fibroblasts.

III. Effect of the active ingredient according to the invention on epigenetic factors

The effect of the active ingredient according to the invention on epigenetic factors was evaluated using a study on older human fibroblasts, by evaluating the expression of SIRT1.
Sirtuin 1 is a deacetylase whose expression is associated with an increase in longevity. Indeed, owing to the deacetylation of the DNA histones, SIRT1 would stabilize the chromatin. It also conditions the expression of certain genes, in particular that of p53, a protein having a negative impact on cell longevity. SIRT1 is described to block p53.

The aim of this study is to determine the capacity of the active ingredient according to the invention to increase SIRT1 expression in older skin cells.

The operating protocol is described as follows:
Normal human fibroblasts from young donors (21 years old) or older donors (68 years old) are cultivated in a culture medium containing little or no active ingredient according to the invention.
After at least one day of contact, the fibroblasts are collected and the whole RNAs are extracted. The mRNAs of SIRT1 are analyzed. The quantification of the florescence incorporation is measured continuously using a thermocycler and the relative quantification is done using software.

The results are summarized in Table 13.

**[Table 13]**

| | **Expression of SIRT1 (%)** | **Ability to restore SIRT1 expression (%)** |
|---|---|---|
| **Young fibroblasts** | | |
| Control | 100 | |
| **Older fibroblasts** | | |

| | | |
|---|---|---|
| Control | 88 | |
| Active ingredient of example 1 0.25% | 145 | +475% |

One can see that the capacity of the older fibroblasts to express SIRT1 is decreased by 12% relative to young fibroblasts.
Tested at 0.25%, the active ingredient according to the invention increases the expression of SIRT1 by 475% in the older fibroblasts.

### IV. Effect of the active ingredient according to the invention on cell detoxification

### IV.1. Effect of the active ingredient according to the invention on the expression of anti-radical enzymes

The effect of the active ingredient according to the invention on the expression of anti-radical enzymes was evaluated using a study on older human fibroblasts, by evaluating the expression of SOD1, thioredoxin and catalase.
SOD1, or superoxide dismutase 1, is an anti-radical enzyme responsible for breaking down superoxide radicals. Catalase makes it possible to break down hydrogen peroxide. Thioredoxin promotes the regeneration of oxidized thiols of proteins, and that of vitamin C.

The operating protocol is described as follows:
The purpose of this study is to determine the capacity of the active ingredient according to the invention to increase SOD1, thioredoxin and catalase expression in older skin cells.
Normal human fibroblasts from older donors (68 years old) are cultivated in a culture medium containing little or no active ingredient according to the invention.
After at least one day of contact, the fibroblasts are collected and the whole RNAs are extracted. The mRNAs of SOD1, thioredoxin and catalase are analyzed in parallel with the mRNAs of RSP18. The quantification of the florescence incorporation is measured continuously using a thermocycler and the relative quantification is done using software.

The results are summarized in Table 14.

**[Table 14]**

| | **Expression of SOD1 (%)** | **Increased expression of SOD1 (%)** | **Expression of vimentin (%)** | **Increased expression of thioredoxin (%)** | **Expression of catalase (%)** | **Increased expression of catalase (%)** |
|---|---|---|---|---|---|---|
| **Older fibroblasts** | | | | | | |
| Control | 100 | | 100 | | 100 | |
| Active ingredient of example 1 0.25% | 115 | +15% | 126 | +26% | 113 | +13% |
| Active ingredient of example 1 1% | 122 | +22% | 142 | +42% | 120 | +20% |

Tested at 1%, the active ingredient according to the invention increases the expression of SOD1, thioredoxin and catalase respectively by 22%, 42% and 20% in the older fibroblasts.

### IV.2. Effect of the active ingredient according to the expression of stress response factors

The effect of the active ingredient according to the invention on the expression of stress response factors was evaluated using a study on older human fibroblasts, by evaluating the expression of HSP72, HSP27 and HSP90.
The aim of this study is to determine the capacity of the active ingredient according to the invention to increase HSP72, HSP27 and HSP90 expression in older skin cells.

The operating protocol is described as follows:
Normal human fibroblasts from young donors (21 years old) and older donors (68 years old) are cultivated in a culture medium containing little or no active ingredient according to the invention.
After at least one day of contact, the fibroblasts are collected and the whole RNAs are extracted. The mRNAs of HSP72, HSP27 and HSP90 are analyzed in parallel with the mRNAs of a housekeeping gene (RSP18). The quantification of the florescence incorporation is measured continuously using a thermocycler and the relative quantification is done using software.

The results are summarized in Table 15.

**[Table 15]**

| | **Expression of HSP72 (%)** | **Capacity to restore HSP72 expression (%)** | **Expression of HSP27 (%)** | **Capacity to restore HSP27 expression (%)** | **Expression of HSP90 (%)** | **Capacity to restore HSP90 expression (%)** |
|---|---|---|---|---|---|---|
| **Young fibroblasts** | | | | | | |
| Control | 100 | | 100 | | 100 | |

| **Older fibroblasts** | | | | | | |
|---|---|---|---|---|---|---|
| Control | 85 | | 78 | | 65 | |
| Active ingredient of example 1 at 0.25% | 104 | +127% | 86 | +36% | 82 | +49% |

One can see that the capacity of the older fibroblasts to express HSP72, HSP27 and HSP90 is decreased by 15%, 22% and 35%, respectively, relative to young fibroblasts. Tested at 0.25%, the active ingredient according to the invention increases the expression of HSP72, HSP27 and HSP90 by 127%, 36% and 49%, respectively, in the older fibroblasts.

### V. Effect of the active ingredient according to the invention on skin inflammation

The effect of the active ingredient on skin inflammation was evaluated through two studies done on human keratinocytes cultures evaluating the synthesis of prostaglandins PGE2 and interleukins IL-8.
This effect was able to be compared to the extract taken from red flowers.

### V.1. Effect of the active ingredient on the release of PGE2

The aim of this study is to determine the capacity of an active ingredient according to the invention or the red flower extract to decrease the inflammation caused by an attack by arachidonic acid by measuring the released prostaglandin E2 (PGE2) level.

The study is carried out on normal human keratinocytes according to the operating protocol described as follows:
On D0, the human keratinocytes are seeded in a complete medium. The cells are next incubated at 37°C.
After several days of culture, the cells are or are not pretreated with a solution of arachidonic acid for 3 hours with or without the presence of the active ingredient according to the invention or the red flower extract.
Next, the cell supernatants are recovered. The assay of the PGE2 is done using an ELISA assay kit.

The results are summarized in Table 16.

**[Table 16]**

| | **PGE2 (pg/mg of proteins)** | **Efficacy/attacked control (%)** |
|---|---|---|
| **Normal keratinocytes** | | |
| Control | 76 | |

| **Keratinocytes attacked by arachidonic acid** | | |
|---|---|---|
| Control | 1783 | |
| Extract of example 1 (white to light pink flowers) 0.25% | 1068 | -40% |
| Extract of red flowers (example 4) 0.25% | 1299 | -27% |

During the inflammation caused by the arachidonic acid, the human keratinocytes secrete prostaglandins E2 (PGE2).
The active ingredient according to the invention decreases the release of PGE2s by the cells attacked by arachidonic acid. Tested at 0.25%, the active ingredient according to the invention reduces the released PGE2 content by 40%, while the red flower extract has a lower PGE2 inhibiting effect.

### V.2. Effect of the active ingredient on the release of interleukins IL-8

The purpose of this study is to determine the capacity of an active ingredient according to the invention or the red flower extract to decrease the inflammation caused by an attack by measuring the released interleukins 8 (IL-8) level. The inflammation is caused by UVB stress.

The study is carried out on normal human keratinocytes according to the operating protocol described as follows:
On D0, the human keratinocytes are seeded in a complete medium. The cells are next incubated at 37°C.
After several days of culture, the cells are or are not radiated with UVB with or without the presence of the active ingredient according to the invention or the red flower extract. Next, the cell supernatants are recovered. The assay of the IL-8 is done using an ELISA assay kit.

The results are summarized in Table 17.

**[Table 17]**

| | **IL-8 (pg/mg of proteins)** | **Efficacy/attacked control (%)** |
|---|---|---|
| **Normal keratinocytes** | | |
| Control | 75 | |

| **UVB-attacked keratinocytes** | | |
|---|---|---|
| Control | 2069 | |
| Active ingredient of example 1 0.25% | 1307 | -37% |
| Extract of example 4 (red flowers) 0.25% | 1503 | -27% |

During inflammation caused by an attack, the human keratinocytes secrete interleukins IL-8.
Tested 0.25% on cells stressed by UVBs, the active ingredient according to the invention induces a greater decrease (-37%) of the released IL-8 level than with the red flower extract (-27%).

Advantageously, the active ingredient according to the invention, derived from white to light pink flowers, is therefore capable of reducing the skin inflammation. It decreases the release of PGE2s and interleukins IL-8.

### V.3. Effect of the active ingredient according to the invention on the skin immune barrier

The effect of the active ingredient according to the invention or the red flower extract on the skin immune barrier was evaluated through a study done on human keratinocytes cultures by measuring the expression of TLR-2 receptors and hBD2 antimicrobial peptides.
This effect was able to be compared to the extract taken from red flowers.
The TLR-2 receptor is described as an innate immunity receptor, while β-defensin 2 (hBD2) acts as an antimicrobial peptide of the skin.

The study was carried out on normal human keratinocytes according to the operating protocol described as follows.
On D0, the human keratinocytes are seeded in a complete medium and the cells are incubated at 37°C.
Next, the cells are treated. The culture medium is eliminated and replaced by medium containing the active ingredient according to the invention or the red flower extract.
The cells are next incubated for 24 hours at 37°C.
After the treatment, the cells are recovered and the whole RNAs are extracted. The RNAs are reverse-transcripts and the obtained complementary DNAs are analyzed by quantitative PCR. The quantification of the florescence incorporation is measured continuously using a thermocycler and the relative quantification is done using software.

The obtained results are shown in Table 18.

**[Table 18]**

| | | **Expression of TLR2 (%)** | **Efficacy/ Control (%)** | **Expression of HBD2 (%)** | **Efficacy/ Control (%)** |
|---|---|---|---|---|---|
| Control | | 100 | | 100 | |
| Active ingredient of example 1 (white to light pink flowers) | 0.1% | 119 | +19% | 125 | +25% |
| | 0.5% | 124 | +24% | 137 | +37% |
| Extract of example 4 (red flowers) | 0.1% | 112 | +12% | 101 | +1% |
| | 0.5% | 112 | +12% | 118 | +18% |

One can see that the active ingredient according to the invention stimulates the expression of the TLR2 receptors and hBD2.

Tested at 0.5% on normal human keratinocytes, the active ingredient according to the invention stimulates the expression of TLR2 by 24% and of hBD2 by 37%. In comparison, the red flower extract only stimulates TLR2 expression by 12% and hBD2 expression by 18%.

Advantageously, the active ingredient according to the invention, derived from white to light pink flowers, is therefore capable of stimulating the immune defenses of the skin. In particular, when it is applied on older skin, it increases the expression of the TLR2 receptors and hBD2 defensins.

Thus, the active ingredient according to the invention, derived from white to light pink flowers, is capable of strengthening the immune barrier twice as much as the red flower extract.

## Claims

1. A cosmetic active ingredient comprising at least one extract obtained from *Hibiscus sinensis* flowers, said extract comprising at least 2 ppm of N-(1-deoxy-1-fructosyl)-proline and/or at least 145 ppm of sarmentosin epoxide.

2. The cosmetic active ingredient according to the preceding claim, wherein the extract is obtained from white flowers and/or light pink flowers of *Hibiscus sinensis.*

3. The cosmetic active ingredient according to one of the preceding claims, wherein the extract is a hydroglycolic extract of *Hibiscus sinensis.*

4. The cosmetic active ingredient according to one of the preceding claims, wherein the extract comprises an isomeric of citric acid with a molar mass of 192 Da, preferably the extract comprises less than 60 ppm of an isomeric of citric acid with a molar mass of 192 Da.

5. The cosmetic active ingredient according to one of the preceding claims, wherein the extract comprises at least 50% carbohydrates by dry weight of the extract, preferably at least 30% by dry weight of the carbohydrates present in the extract are oligosaccharides and polysaccharides with molar masses of between 360 and 1,620 Da.

6. The cosmetic active ingredient according to one of the preceding claims, wherein the extract comprises minerals and/or proteins, preferably the extract comprises less than 20% minerals and/or less than 20% proteins.

7. The cosmetic active ingredient according to the preceding claim, wherein the proteins are peptides with molar masses below 2,000 Da.

8. A method for obtaining an extract according to one of the preceding claims, wherein it comprises a step for solubilization in a water/butylene glycol mixture, of *Hibiscus sinensis* flowers in order to obtain an extract comprising at least 2 ppm of N-(1-deoxy-1-fructosyl)-proline and/or at least 145 ppm of sarmentosin epoxide.

9. The method according to the preceding claim, wherein it comprises carrying out the following steps:
- solubilizing the flower powder in a water/butylene glycol mixture,
- separating the soluble and insoluble phases, to recover the soluble phase,
- filtrations,
- sterilizing filtration.

10. A cosmetic use for topical application on the skin of a cosmetic active ingredient, wherein the active ingredient is the active ingredient according to one of claims 1 to 7, or a composition containing said active ingredient.

11. The use according to the preceding claim, to prevent and/or combat skin aging.

12. A cosmetic active ingredient according to one of claims 1 to 7 or a composition containing said active ingredient for its use to:
- promote skin regeneration, and/or
- regulate the epigenetic factors responsible for skin aging, and/or
- increase cell detoxification of the skin, and/or
- limit skin inflammation, and/or
- strengthen the skin immune barrier.

13. A cosmetic composition comprising at least 0.1% by weight of a liquid active ingredient according to one of claims 1 to 7.

14. The cosmetic composition according to the preceding claim, wherein it assumes the form of a gel, emulsion or cream.

15. A cosmetic product for treating the skin for a skin anti-aging effect, wherein it consists of the topical application on the skin of a composition according to one of claims 13 or 14.

## Patentansprüche

1. Kosmetischer Wirkstoff, umfassend mindestens einen Extrakt, der aus *Hibiscus* sinensis-Blüten gewonnen wird, der Extrakt umfassend mindestens 2 ppm N-(1-Desoxy-1-fructosyl)-prolin und/oder mindestens 145 ppm Sarmentosinepoxid umfasst.

2. Kosmetischer Wirkstoff nach dem vorstehenden Anspruch, wobei der Extrakt aus weißen Blüten und/oder hellrosa Blüten von *Hibiscus sinensis* gewonnen wird.

3. Kosmetischer Wirkstoff nach einem der vorstehenden Ansprüche, wobei der Extrakt ein hydroglykolischer Extrakt von *Hibiscus sinensis* ist.

4. Kosmetischer Wirkstoff nach einem der vorstehenden Ansprüche, wobei der Extrakt ein Isomer von Zitronensäure mit einer Molmasse von 192 Da umfasst, vorzugsweise der Extrakt weniger als 60 ppm eines Isomers von Zitronensäure mit einer Molmasse von 192 Da umfasst.

5. Kosmetischer Wirkstoff nach einem der vorstehenden Ansprüche, wobei der Extrakt mindestens 50 % Kohlenhydrate bezogen auf das Trockengewicht des Extrakts umfasst, vorzugsweise mindestens 30 % bezogen auf das Trockengewicht der Kohlenhydrate, die in dem Extrakt vorhanden sind, Oligosaccharide und Polysaccharide mit Molmassen zwischen 360 und 1.620 Da sind.

6. Kosmetischer Wirkstoff nach einem der vorstehenden Ansprüche, wobei der Extrakt Mineralien und/oder Proteine umfasst, vorzugsweise der Extrakt weniger als 20 % Mineralien und/oder weniger als 20 % Proteine umfasst.

7. Kosmetischer Wirkstoff nach dem vorstehenden Anspruch, wobei die Proteine Peptide mit Molmassen unter 2.000 Da sind.

8. Verfahren zum Erhalten eines Extrakts nach einem der vorstehenden Ansprüche, wobei es einen Schritt für eine Solubilisierung in einem Wasser/Butylenglykol-Gemisch von *Hibiscus* sinensis-Blüten umfasst, um einen Extrakt zu erhalten, umfassend mindestens 2 ppm N-(1-Desoxy-1-fructosyl)-prolin und/oder mindestens 145 ppm Sarmentosinepoxid.

9. Verfahren nach dem vorstehenden Anspruch, wobei es das Ausführen der folgenden Schritte umfasst:
- Solubilisieren des Blütenpulvers in einem Wasser/Butylenglykol-Gemisch,
- Trennen der löslichen und unlöslichen Phasen, um die lösliche Phase zu gewinnen,
- Filtrationen,
- sterilisierende Filtration.

10. Kosmetische Verwendung für eine topische Anwendung auf der Haut eines kosmetischen Wirkstoffs, wobei der Wirkstoff der Wirkstoff nach einem der Ansprüche 1 bis 7 ist, oder eine Zusammensetzung, die den Wirkstoff enthält.

11. Verwendung nach dem vorstehenden Anspruch, um Hautalterung vorzubeugen und/oder zu bekämpfen.

12. Kosmetischer Wirkstoff nach einem der Ansprüche 1 bis 7 oder eine Zusammensetzung, die den Wirkstoff enthält, für dessen Verwendung zum:
- Fördern von Hautregeneration und/oder
- Regulieren der epigenetischen Faktoren, die für Hautalterung verantwortlich sind und/oder
- Verstärken von Zellentgiftung der Haut und/oder
- Begrenzen von Hautentzündungen und/oder
- Stärken der Hautimmunbarriere.

13. Kosmetische Zusammensetzung, umfassend zu mindestens 0,1 Gew.-% einen flüssigen Wirkstoff nach einem der Ansprüche 1 bis 7.

14. Kosmetische Zusammensetzung nach dem vorstehenden Anspruch, wobei sie die Form eines Gels, einer Emulsion oder einer Creme annimmt.

15. Kosmetisches Produkt zum Behandeln der Haut für einen Anti-Hautalterungseffekt, wobei es aus der topischen Anwendung auf der Haut einer Zusammensetzung nach einem der Ansprüche 13 oder 14 besteht.

## Revendications

1. Principe actif cosmétique comprenant au moins un extrait obtenu à partir de fleurs d'*Hibiscus sinensis,* ledit extrait comprenant au moins 2 ppm de N-(1-désoxy-1-fructosyle)-proline et/ou au moins 145 ppm d'époxyde de sarmentosine.

2. Principe actif cosmétique selon la revendication précédente, dans lequel l'extrait est obtenu à partir de fleurs blanches et/ou de fleurs rose clair d'*Hibiscus sinensis.*

3. Principe actif cosmétique selon l'une des revendications précédentes, dans lequel l'extrait est un extrait hydroglycolique d'*Hibiscus sinensis.*

4. Principe actif cosmétique selon l'une des revendications précédentes, dans lequel l'extrait comprend un isomère d'acide citrique avec une masse molaire de 192 Da, de préférence l'extrait comprend moins de 60 ppm d'un isomère d'acide citrique avec une masse molaire de 192 Da.

5. Principe actif cosmétique selon l'une des revendications précédentes, dans lequel l'extrait comprend au moins 50 % d'hydrates de carbone en poids sec de l'extrait, de préférence au moins 30 % en poids sec des hydrates de carbone présents dans l'extrait sont des oligosaccharides et des polysaccharides avec des masses molaires comprises entre 360 et 1 620 Da.

6. Principe actif cosmétique selon l'une des revendications précédentes, dans lequel l'extrait comprend des minéraux et/ou des protéines, de préférence l'extrait comprend moins de 20 % de minéraux et/ou moins de 20 % de protéines.

7. Principe actif cosmétique selon la revendication précédente, dans lequel les protéines sont des peptides avec des masses molaires inférieures à 2 000 Da.

8. Procédé pour l'obtention d'un extrait selon l'une des revendications précédentes, dans lequel il comprend une étape de solubilisation dans un mélange eau/butylène glycol, de fleurs d'*Hibiscus sinensis* afin d'obtenir un extrait comprenant au moins 2 ppm de N-(1-désoxy-1-fructosyle)-proline et/ou au moins 145 ppm d'époxyde de sarmentosine.

9. Procédé selon la revendication précédente, dans lequel il comprend la réalisation des étapes suivantes :
- solubilisation de la poudre de fleur dans un mélange eau/butylène glycol,
- séparation des phases soluble et insoluble, pour récupérer la phase soluble,
- filtrations,
- filtration stérilisante.

10. Utilisation cosmétique pour une application topique sur la peau d'un principe actif cosmétique, dans laquelle le principe actif est le principe actif selon l'une des revendications 1 à 7, ou une composition contenant ledit principe actif.

11. Utilisation selon la revendication précédente, pour prévenir et/ou combattre le vieillissement de la peau.

12. Principe actif cosmétique selon l'une des revendications 1 à 7 ou composition contenant ledit principe actif pour son utilisation pour :
- favoriser la régénération de la peau, et/ou
- réguler les facteurs épigénétiques responsables du vieillissement de la peau, et/ou
- augmenter la détoxication cellulaire de la peau, et/ou
- limiter l'inflammation de la peau, et/ou
- renforcer la barrière immunitaire de la peau.

13. Composition cosmétique comprenant au moins 0,1 % en poids d'un principe actif liquide selon l'une des revendications 1 à 7.

14. Composition cosmétique selon la revendication précédente, dans laquelle elle prend la forme d'un gel, d'une émulsion ou d'une crème.

15. Produit cosmétique destiné au traitement de la peau pour un effet anti-âge, dans lequel il consiste en l'application topique sur la peau d'une composition selon l'une des revendications 13 ou 14.
